Europäisches Patentamt

(19) European Patent Office (11) Publication number: **0 065 370**

Office européen des brevets **B1**

(12) **EUROPEAN PATENT SPECIFICATION**

(45) Date of publication of patent specification: **23.01.85**

(21) Application number: **82302204.1**

(22) Date of filing: **29.04.82**

(54) Sterile dressing and preparation thereof.

(30) Priority: **02.05.81 GB 8113624**

(43) Date of publication of application:
**24.11.82 Bulletin 82/47**

(45) Publication of the grant of the patent:
**23.01.85 Bulletin 85/04**

(84) Designated Contracting States:
**BE CH DE FR IT LI NL SE**

(56) References cited:
**EP-A-0 020 777**
**GB-A-1 108 837**
**US-A-3 328 259**
**US-A-3 598 122**
**US-A-4 197 289**

(73) Proprietor: **Smith and Nephew Associated
Companies p.l.c.
2, Temple Place Victoria Embankment
London WC2R 3BP (GB)**

(72) Inventor: **Tharratt, John
73 Graham Avenue Hessle Road
Hull (GB)**
Inventor: **Harlock, Sally-Jane
562 Holderness Road
Hull HU9 3ET (GB)**

(74) Representative: **Cole, William Gwyn
Corporate Patents Department Smith and
Nephew Research Limited Gilston Park
Harlow Essex CM20 2RQ (GB)**

Courier Press, Leamington Spa, England.

# Description

This invention relates to a sterile dressing suitable for use on wounds and to its preparation. More particularly this invention relates to a sterile dressing of the type comprising a water soluble conformable sheet which when placed on a moist wound dissolves to release a medicament and to the preparation of such a dressing.

Topical creams containing medicaments are often applied to wounds but these are normally applied by rubbing with a gloved hand or by spreading with a pallet knife which can cause pain particularly in large wounds and especially with burns. Medicated occlusive dressing have also been widely used but these also have the disadvantages of being difficult to remove, do not permit sufficient air contact with the wound and may cause maceration of healing tissue. Further, an indeterminate amount of medicament agent is applied to the wound.

In an attempt to overcome the problems of using creams in general, dressings applicable in the form of a water soluble sheet material have been described. US Patent No. 3,328,259 describes the use of certain water soluble cellulose derivatives as wound dressings. However preparing such dressings in sterile form is difficult unless full aseptic assembly is envisaged.

British Patent No. 1,471,013 and US Patent No. 3,969,498 teach the use of a dextran polymer to provide an aerated, water soluble plasma soluble dressing containing a medicament agent. However it was not said to be possible to prepare a sterile dressing by terminal sterilisation. The inventors of the aforementioned patents have subsequently described how their invention may be used to prepare effective dry foams (see Journal of Pharmaceutical Sciences 1974 *63* (9) p.1483—4).

British Patent No. 1521171 discloses the use of an insoluble acrylonitrile-dimethylaminoethyl methacrylate copolymer membrane with a medicament dispersed in it as a burn wound covering. Whilst this membrane is permeable to water vapour and air it is not soluble and hence will not rapidly release medicament when placed on the moist wound.

Such known dressings have apparently been difficult to provide in sterile form and it is now thought that this in many cases is due to degradation of the medicament or film material on sterilisation.

British Patent No. 1108837 discloses water soluble films in which a local anaesthetic agent is finely distributed within one layer while the other layer is free thereof. No suggestion was made that the local anaesthetic could be anywhere other than distributed within one of the layers. United States Patent Nos. 4,128,445 and 4,197,289 disclose oral dosage units comprising two layers of an edible, water soluble or water dispersible web laminated together in which the medicament is loaded onto an internal surface of one of the layers. However, there is no disclosure of sterile laminates of this type or to their use as wound dressings.

Clearly it would be of advantage to provide a sterile, water soluble, conformable dressing which would dissolve to release medicament when placed on a moist wound which dressing could be prepared without recourse to full aseptic assembly techniques and which would be adapted to employ a number of different medicaments. Such a dressing has now been discovered.

The present invention provides a water-soluble, conformable wound dressing which can dissolve to release medicament when placed on a moist wound characterised in that said dressing comprises a sterile laminate of two sheets of water soluble film forming material between which is located the medicament.

The film forming materials employed in this invention may be any pharmaceutically acceptable material which forms water-soluble, conformable sheets. Suitable materials include polyvinyl alcohols, polyvinyl pyrrolidone, sodium carboxymethylcellulose, hydroxypropylcellulose, methyl-cellulose, ethylcellulose or mixtures thereof or other water soluble materials such as those set forth in the aforementioned patents.

Preferred film forming material includes polyvinyl alcohol. Herein the term polyvinyl alcohol is included not only polyvinyl alcohol per se but partially acetylated polyvinyl alcohol. Aptly the polyvinyl alcohol employed in this invention contains 10% to 33% of acetylated hydroxyl groups, more suitably 15% to 25% of acetylated hydroxyl groups and preferably 10% to 22% of acetylated hydroxyl groups. Aptly the polyvinyl alcohol employed in this invention has a molecular weight of from 15,000 to 120,000, more suitably from 20,000 to 100,000, and preferably from 25,000 to 85,000.

Apt polyvinyl alcohols are Gohsenol (Trade Mark) GM13 and Gohsenol GL05 made by Nippon Gohsei and available from British Traders and Shippers Limited. Gohsenol GM14 has a molecular weight of 83,000 and a degree of hydrolysis of 87% to 89%. Gohsenol GL05 has a molecular weight of 30,000 and a degree of hydrolysis of 87% to 89%.

Gohsenol GL05 is the preferred polyvinyl alcohol.

The water soluble sheet material will generally contain from 1 to 50% of plasticizer, more suitably from 5 to 40% of plasticizer and favourably from 10 to 25% of plasticizer.

The plasticizer employed in the sheet material will normally be water, an alcohol or mixture thereof. Frequently the sheet material will contain up to 25% water, more usually 1 to 20% water and commonly 2 to 10% water. The alcohol employed will usually be a di- or tri-hydroxylic compound such as ethylene glycol, propylene glycol, tetramethylene glycol, glycerol or the like. Favoured alcohols include propylene glycol and glycerol of which glycerol is normally preferred. Frequently the sheet material will contain 1 to 25% of the alcohol, more usually 1 to 20% of the alcohol and favourably 8 to 15% of the alcohol.

Suitable sheets of film forming material can have a weight per unit area of 10 to 125 g/m² and preferably a weight per unit area of 15 to 50 g/m².

The sheet of film forming material can be a film of substantially uniform thickness or a film with thinner areas. Suitable films can have a thickness of 10 to 25 $\mu$m and preferably a thickness of 12.5 to 50 $\mu$m.

A favoured sheet of film forming material is polyvinyl alcohol (Gohsenol GL05) film of approximately 25 micron thickness containing 5 to 15% by weight of a glycerol plasticiser.

However in another aspect at least one of the sheets of the sterile laminate of the invention can have thinner areas to aid dispersal of the sheet in water such thinner areas are preferably in the form of recesses arranged in a uniform pattern on one or both surfaces of the sheet. Suitable recesses include grooves which may be interconnected, for example intersecting sets of straight parallel grooves arranged in a grid pattern and depressions, which may be discrete, of for example triangular, rectangular, circular or a like geometric shape.

A favoured sheet of film forming material having thinner areas is a polyvinyl alcohol (Gohsenol GL05) film (weight per unit area of approximately 24 g/m²) containing 5% to 15% be weight of glycerol with a uniform pattern of triangular shaped depressions on one surface.

The use of such patterned films can reduce the 'blocking' of the laminate surfaces with other layers for example during production or within the pack.

The medicament employed is normally a curative agent which is topically effective. One particular class of curative agents envisaged for use in this invention are the topically applicable anti-infective agents. Such agents include chlorhexidine salts such as the gluconate, acetate, hydrochloride or the like; silver salts such as silver sulphadiazine; iodophors such as polyvinylpyrrolidone-iodine or the like; or other topically acceptable antibacterial agent. Other topically acceptable medicaments include antiinflammatory agents such as steroids, for example cortisone, hydrocortisone, fludrocortisone, dexamethasone, fluoromethalone, prednisolone, triamcinolone, betamethasone, flumethasone, fluocinolone, methylprednisolone, trimeloxone, medrysone, hydrocortisone acetate, betamethasone valerate, betamethasone propionate, betamethasone benzoate, fluocinolone acetate, triamcinolone acetonide, prednisolone acetate or the like. Preferred medicaments for use in this invention are povidone iodine, chlorhexidine acetate and silver sulphadiazine.

It is preferred that the medicament is a powder. Suitable powders have a mean particle size of 1 micron to 500 microns and preferably have a mean particle size of 2 $\mu$m to 300 $\mu$m, for example 5 $\mu$m to 250 $\mu$m. Granulated powders can also be used.

Favoured povidone iodine powders include grades 30/06 and 17/12 manufactured by BASF and presently available from Blagden Chemicals Limited. Grades 30/60 and 17/12 have a mean particle size of between 10 and 200 microns, Grade 30/06 has an average molecular weight of 40,000 and an available $I_2$ contect of 1 to 12% and Grade 17/12 has an average molecular weight of 12,000 and an available $I_2$ content of 9 to 12%. Micronised powders of these grades having a particle size of less than 10 microns are also suitable.

A favoured chlorhexidine acetate powder has a mean particle size of 10 $\mu$m.

A favoured silver sulphadiazine powder has a mean particle size of 2 to 5 $\mu$m.

As previously indicated the medicament will be located between the soluble sheets of film forming material. Normally the medicament will form 0.1% to 25% of the dressing and more usually from 0.5 to 15% of the dressing.

Generally antibacterial agents will be present by at least 0.1% and usually be at least 2% of the dressing. Generally the steroids will be present by not more than 10% and usually not more than 2% of the dressing.

It is desirable that the medicament is distributed evenly over the operative area of the dressing. This may be done most easily by an even distribution of spots, lines or other pattern of medicament. A continuous coating over the operative area of the dressing may also be employed especially if said operative area does not comprise the whole of the dressing.

The medicament may be located on the surface of a flat sheet of film forming material or on the surface of indentations in an indented sheet of film forming material.

The actual pattern of medicament is relatively unimportant as long as it achieves an even distribution of medicament over the wound when the dressing dissolves.

Often the medicament will be located on 5 to 80% of the surface area of the sheet, more usually 10 to 70% favourably 20 to 60% and preferably 25 to 50% of the surface area of the sheet. The medicament will disperse throughout the wound on dissolution of the film forming material.

Methods of locating the medicament include printing onto a flat surface, filling into an indented surface, distributing over a flat surface using a template having a pattern of holes or the like.

A favoured method of locating the medicament comprises printing a sheet of film forming material with a uniform pattern of spots of contacting the sheet against a roller having a corresponding pattern of depressions on its circumferential surface filled with medicament powder. The roller depressions can be conveniently filled by means of coating head trough.

Preferably the roller surface has a pattern of depressions or a series of repeat patterns of depressions of a size which matches that of the desired dressing.

In the case of potent medicaments of acceptable diluent may be included if desired.

Suitale water soluble polymer films can be made by hot melt extrusion or by casting an aqueous solution of the polymer onto a release surface or the like. Polyvinyl alcohol films can be conveniently made by casting a 4% to 35% usually 5% to 20% by weight aqueous solution of the polymer in conventional manner for example by means of a doctor blade over flat bed coating head onto a polyethylene coated paper and then drying in an oven at approximately 85°C. A favoured polyethylene coated paper is known as Steralease 15 (Trade Mark) made by Sterling Coated Papers Limited.

Suitable water soluble films having thinner areas can be made by casting the polymer, as a solution or a hot melt, against a surface having a pattern of raised areas. These films can also be made heat embossing a plain film with similar patterned surface.

An apt process of forming such films comprises casting an aqueous solution of polymer in contact with a sheet embossed with uniform pattern or raised areas.

A favoured process of forming a polyvinyl alcohol film with thinner areas comprises casting an aqueous solution of polyvinyl alcohol (30% by weight of Gohsenol GL05) containing a plasticiser (5% to 15% by weight of glycerol) by means of doctor blade over flat bed coating head onto a film embossed with a uniform pattern of raised triangles (polyethylene film type 04514 emboss code 14 available from A O E Plastics GmbH).

In another aspect the invention provides a process of making a sterile laminate of the invention which comprises laminating two sheets of water soluble film forming material with a medicament located between said sheets.

The two sheets of film forming material in the sterile laminates of the invention can be bonded together over substantially the whole of their surfaces. However, it is preferred that the medicament is located in unbonded areas of the laminate.

In a preferred aspect of the sterile laminates of the invention the medicament is located in discrete areas between bonded areas of the laminate. Such bonding prevents the medicament moving to other areas of the laminate and thus maintains the even distribution of the medicament over the operative area of the dressing.

A favoured sterile laminate of the invention has a uniform pattern of spots in medicament at unbonded areas of the laminate located between a pattern of square grid bonded areas.

The two sheets of film forming material may be laminated together in any convenient manner, for example by solvent welding (for example with a plasticizer as hereinbefore described), heat sealing, pressure sealing, or adhesive bonding. One of the great advantages of using polyvinyl alcohol as the film forming material is that it has now been found possible to pressure seal the two layers together to form a laminate. Suitable adhesive include pressure sensitive adhesives, water and solvent based adhesives and hot melt adhesives. Preferred adhesives are water soluble or water dispersible pressure sensitive adhesive.

A preferred method of forming a sterile laminate of the invention comprises printing a film of polyvinyl alcohol with a thin layer of water and laminating the printed film under pressure to a film of polyvinyl alcohol with medicament thereon.

Desirably during the laminating process the relative arrangement of the two polyvinyl alcohol films is such that the water printed areas on one film does not coincide with medicament coated areas on the other film so that the medicament is located in discrete areas between bonded areas of the laminate.

In a favoured process a film of polyvinyl alcohol is printed with a square grid pattern of water and laminated under pressure to a film of polyvinyl alcohol coated with a uniform pattern of medicament spots so that spots of medicament are located within square grid bonded areas of the laminate. The polyvinyl alcohol film can be printed with a square grid pattern of water by contacting the film under pressure against a roller having a corresponding square grid pattern of flat topped ridges on its circumferential surface which are supplied with a thin layer of water by a conventional series contra-rotating feed rollers from a water trough.

The dressing of this invention will generally be from 40 to 250 $\mu$m, more usually 75 to 150 $\mu$m and preferably 80 to 120 $\mu$m, for example 100 $\mu$m thick. The two layers of film forming materials will generally be about half the aforementioned thicknesses.

In a continuous process the laminate can be formed into strip which can be cut into dressings of suitable size.

The dressings can be individually packed into sealed bacteria-proof packs. Suitable packs include pouches made of paper or like ethylene oxide permeable material and pouches made of aluminium foil laminates.

Such aluminium foil laminate pouches can be made by heat sealing two layers of a suitable aluminium foil laminate in a conventional manner.

Suitable heat sealable aluminium foil laminate for example a paper/aluminium foil/ ethylene vinyl acetate lacquer laminate are available from DGR Flexible Packaging Limited. The laminate dressing can be sterilised within the bacteria-proof pack to form the sterile laminate of the invention.

The laminate dressing within the bacteria-proof pack can be sterilised by ethylene oxide (when the pack is permeable to ethylene oxide) or gramma irradiation in a conventional manner.

The dressings will have a suitable size and shape for the treatment of wound areas. Suitably dressings have a rectangular shape and are from 5 cm to 10 cm in the length or width dimension, typically 10 cm×10 cm, 10 cm×7.5 cm, and 5 cm×5 cm.

The separation of the medicament from the matrix of the sheets of film forming material reduces interaction of the components which would otherwise occur on sterilisation. As previously indicated this allows a number of medicaments and film forming substances to be employed in combination which would not otherwise be practicable. A further advantage of this separation of the components of the dressing in this manner is that each of the components can be sterilised separately in conventional manner and the dressing fabricated under aseptic conditions during the last stages only rather than having to carefully maintain sterility at all stages of manufacture. Many of the dressings of this invention can be terminally sterilised by 2.5 Mrad radiation which is yet another advantage.

The following Examples illustrate the invention.

Example 1

A 10% by weight aqueous solution of polyvinyl alcohol (Gohsenol GM14) was cast onto a polyethylene coating paper (Steralease 15) by a doctor blade over flat bed coating head and dried at 85°C for 5 minutes in an oven to give a 25 micron thick 10 cm wide strip of polyvinyl alcohol film with a residual water content of approximately 6%. The upper surface of the film had a glossy surface. Micronised chlorhexidine acetate (mean particle size 100 microns) was distributed on to the glossy surface of a 10 cm wide strip of polyvinyl alcohol film as a spaced series of 4×3 pattern of dots 1 cm in diameter and 1 cm apart by means of a plastic template having a pattern of approximately circular holes 1 cm in diameter. A 10 cm wide strip of 35 micron thick polyvinyl alcohol film (glossy surface) was then laminated to the chlorhexidine acetate coated film by passage through the nip of rubber rollers under pressure. The strip was cut into 10 cm×8 cm dressing which were packed into sealed aluminium foil pouches and sterilised by gamma irradiation (2.5 mega rads) in a conventional manner.

The water soluble dressing contained approximately 0.07 grams of chlorhexidine acetate fixed in place between two sealed polyvinyl alcohol films as a pattern of 4×3 dots 1 cm in diameter and 1 cm apart with a $1\frac{1}{2}$ cm sealed medicament free margin around the edge of the dressing.

Example 2

A soluble film laminate dressing was made in the same manner as Example 1 using povidone iodine powder (mean particle size 200 microns) instead of chlorhexidine acetate.

Example 3

A soluble film laminated dressing was made in the same manner as Example 1 using micronised silver sulphadiazine (mean particle size 2 $\mu$m to 5 $\mu$m) instead of chlorhexidine acetate.

Example 4

A 10 cm wide strip of polyvinyl alcohol film (thickness 25 microns) containing a glycerol plasticiser (10% by weight) was prepared in the same manner as example 1 using a 30% aqueous solution of polyvinyl alcohol (Gohsenol GL05).

Povidone iodine powder (grade 30/06) was printed onto the glossy surface of the polyvinyl alcohol strip in the form of spots by pressure contact with a printing roller. The printing roller had a repeat pattern of 9×7 square shallow depressions (0.7 cm square) arranged in parallel rows (centres 1 cm apart) on its circumferential surface. The print roller, its depressions filled with powder by means of a coating head, transferred a corresponding pattern of spots onto the polyvinyl alcohol strip.

A second 10 cm wide strip of polyvinyl alcohol, printed with water in a square grid pattern (1 cm squares), was laminated under pressure by nip rollers to the coated polyvinyl alcohol strip so that the povidone-iodine powder was located between the two strips. The water print roller had a square grid pattern (1 cm square) of flat topped ridges which were coated with a thin layer of water by contact with rotating roller which in turn was in contact with contra-rotating water pick up roller partially immersed in a water trough.

The relationship between the printed patterns on polyvinyl alcohol strips before lamination was arranged so that the spots of povidone-iodine powder after lamination were located between bonded areas of the polyvinyl alcohol film laminate strip.

The strip laminate was cut into 10 cm×7.5 cm dressings which were individually packed into sealed aluminium foil laminate pouches and sterilised by gamma irradiation (2.5 megarads) in a conventional manner. The aluminium foil pouches were formed by heat sealing a front foil consisting of a bonded laminate of paper (40 gm/mm²)/aluminium foil (24g/m²)/ethylene-vinyl acetate (23g/m² of Surly 1652 (Trade Mark)) and a back aluminium foil laminate of similar construction coated with a heat seal peelable lacquer (25 g/m²) available from DRG flexible Packaging Limited.

The water soluble dressing contained approximately 10% by weight of povidone iodine located between the polyvinyl alcohol films as a pattern of 7×9 spots arranged in parallel rows (centres approximately 1 cm apart), surrounded by a grid pattern of bonded areas of the laminate.

## Claims

1. A water soluble conformable wound dressing which can dissolve to release medicament when placed on a moist wound characterised in that said dressing comprises a sterile laminate of two sheets of water soluble film forming material between which is located the medicament.

2. A sterile laminate as claimed in claim 1 within a sealed bacteria proof pack.

3. A sterile laminate as claimed in either of claims 1 or 2 in which the film forming material comprises a polyvinyl alcohol containing 10% to 22% acetylated hydroxyl groups.

4. A sterile laminate as claimed in any of claims 1 to 3 in which the medicament is a powder.

5. A sterile laminate as claimed in any of claims 1 to 4 in which the medicament comprises a topically acceptable antibacterial agent.

6. A sterile laminate as claimed in claim 5 in which the antibacterial agent is a chlorhexidine salt.

7. A sterile laminate as claimed in claim 5 in which the antibacterial agent is polyvinyl pyrrolidone iodine.

8. A sterile laminate as claimed in any of claims 1 to 7 in which the medicament forms 0.5% to 15% by weight of the dressing.

9. A sterile laminate as claimed in any of claims 1 to 8 in which the medicament is distributed evenly over the operative area of the dressing.

10. A sterile laminate as claimed in any of claims 1 to 9 in which the two sheets of the laminate are bonded together.

11. A sterile laminate as claimed in claim 10 in which the medicament is located in discrete areas between bonded areas of the laminate.

12. A sterile laminate as claimed in any of claims 1 to 11 in which the dressing has a thickness of 75 to 150 $\mu$m.

13. A sterile laminate as claimed in any of claims 1 to 12 in which at least one of the sheets has thinner areas to aid dispersal of the sheet in water.

14. A process of making a sterile laminate as described in claims 1 to 13 which comprises laminating two sheets of water soluble film forming material with a medicament located between said sheets.

## Patentansprüche

1. Wasserlöslicher, schmiegsamer Wundverband, der sich zur Medikamentfreigabe auflösen kann, wenn er auf eine feuchte Wunde gebracht wird, dadurch gekennzeichnet, daß der Verband ein steriles Laminat aus zwei Bahnen aus wasserlöslichem, filmbildendem Material umfaßt, zwischen denen das Medikament liegt.

2. Steriles Laminat nach Anspruch 1 in einer versiegelten, bakteriendichten Packung.

3. Laminat nach einem der Ansprüche 1 oder 2, worin das filmbildende Material einen Polyvinylalkohol umfaßt, der 10% bis 22% acetylierte Hydroxylgruppen enthält.

4. Steriles Laminat nach einem der Ansprüche 1 bis 3, worin das Medikament ein Pulver ist.

5. Steriles Laminat nach einem der Ansprüche 1 bis 4, worin das Medikament ein topisch annehmbares antibakterielles Mittel umfaßt.

6. Steriles Laminat nach Anspruch 5, worin das antibakterielle Mittel ein Chlorhexidinsalz ist.

7. Steriles Laminat nach Anspruch 5, worin das antibakterielle Mittel Polyvinylpyrrolidon-Jod ist.

8. Steriles Laminat nach einem der Ansprüche 1 bis 7, worin das Medikament 0,5 bis 15 Gew.-% des Verbandes bildet.

9. Steriles Laminat nach einem der Ansprüche 1 bis 8, worin das Medikament gleichmäßig über die Arbeitsfläche des Verbandes verteilt ist.

10. Steriles Laminat nach einem der Ansprüche 1 bis 9, worin die beiden Bahnen des Laminats aneinander gebunden sind.

11. Steriles Laminat nach Anspruch 10, worin das Medikament in diskreten Bereichen zwischen gebundenen Bereichen des Laminats liegt.

12. Steriles Laminat nach einem der Ansprüche 1 bis 11, worin der Verband eine Dicke von 75 bis 150 $\mu$m hat.

13. Steriles Laminat nach einem der Ansprüche 1 bis 12, worin wenigstens eine der Bahnen dünnere Bereiche zur Unterstützung des Zerstreuens der Bahn in Wasser aufweist.

14. Verfahren zur Herstellung eines sterilen Laminats, wie in den Ansprüchen 1 bis 13 beschrieben, das das Laminieren zweier Bahnen aus wasserlöslichem, filmbildendem Material mit einem zwischen den Bahnen befindlichen Medikament umfaßt.

## Revendications

1. Pansement de conformation adaptable soluble dans l'eau, pouvant si dissoudre pour libérer le médicament lorsqu'il est placé sur une plaie humide, caractérisé en ce que ce pansement est constitué par un stratifié stérile formé de deux feuilles de matières filmogène soluble dans l'eau entre lesquelles le médicament est placé.

2. Stratifié stérile suivant la revendication 1, placé dans un emballage scellé étanche aux bactéries.

**0 065 370**

3. Stratifié stérile suivant la revendication 1 ou 2, caractérisé en ce que la matière filmogène est constituée par un alcool polyvinylique contenant de 10 % à 22 % de groupes hydroxyl acétylés.

4. Stratifié stérile suivant l'une quelconque des revendications 1 à 3, caractérisé en ce que le médicament est une poudre.

5. Stratifié stérile suivant l'une quelconque des revendications 1 à 4, caractérisé en ce que le médicament comprend un agent antibactérien acceptable pour applications locales.

6. Stratifié stérile suivant la revendication 5, caracterisé en ce que l'agent antibactérien est un sel de chlorhexidine.

7. Stratifié stérile suivant la revendication 5, caractérisé en ce que l'agent antibactérien est de la polyvinylpyrrolidone-iode.

8. Stratifié stérile suivant l'une quelconque des revendications 1 à 7, caractérisé en ce que le médicament représente de 0,5 % à 15 % en poids du pansement.

9. Stratifié stérile suivant l'une quelconque des revendications 1 à 8, caractérisé en ce que le médicament est distribué uniformément sur la surface active du médicament.

10. Stratifié stérile suivant l'une quelconque des revendications 1 à 9, caractérisé en ce que les deux feuilles du stratifié sont réunies entre elles.

11. Stratifié stérile suivant la revendication 10, caractérisé en ce que le médicament est disposé dans de petites zones placées entre les zones de liaison du stratifié.

12. Stratifié stérile suivant l'une quelconque des revendications 1 à 11, caractérisé en ce que le pansement a une épaisseur allant de 75 à 150 μm.

13. Stratifié stérile suivant l'une quelconque des revendications 1 à 12, caractérisé en ce que l'une au moins des feuilles présente des zones plus minces pour faciliter la dispersion de la feuille dans l'eau.

14. Procédé pour la fabrication d'un stratifié stérile suivant l'une quelconque des revendications 1 à 13, caractérisé en ce qu'on réunit par stratification deux feuilles de matière filmogène soluble dans l'eau avec un médicament placé entre ces feuilles.